# EUROPEAN PATENT SPECIFICATION

(11) **EP 0 726 771 B1**
(45) Date of publication and mention of the grant of the patent: **23.01.2002**
(21) Application number: 94932077.4
(22) Date of filing: 24.10.1994
(51) Int. Cl.: A01N 43/16, A61K 9/16, A61K 31/16, A61K 31/35, A61K 38/23, A61K 38/28

(54) **DESFERRIOXAMINE ORAL DELIVERY SYSTEM**
ORALES VERABREICHUNGSSYSTEM VON DESFERRIOXAMIN
SYSTEME D'ADMINISTRATION ORALE DE DEFEROXAMINE

(30) Priority: 26.10.1993 US 143571; 16.12.1993 US 168776
(43) Date of publication of application: 21.08.1996
(60) Divisional application: 00117452.3
(73) Proprietor: Emisphere Technologies, Inc., Tarrytown, New York 10591 (US)
(72) Inventor: MILSTEIN, Sam J., Larchmont, NY 10538 (US); BARANTSEVITCH, Evgueni N., New Rochelle, NY 10801 (US)
(74) Representative: Grünecker, Kinkeldey, Stockmair & Schwanhäusser Anwaltssozietät
(86) International application number: PCT/US94/12333
(87) International publication number: WO 95/11690

(56) References cited:
- EP-A- 0 068 314
- EP-A- 0 418 642
- WO-A-88/01213
- WO-A-94/23767
- GB-A- 2 095 994
- US-A- 4 925 673
- PEDIATRIC RESEARCH, Volume 23, Number 4, Part 2, issued 1988, WATTERBERG et al., "Aerosolized Cromolyn Sodium Delivery to Intubated Babies", page 570A, column 1, abstract number 2209.
- CHEST, Volume 87, Number 1 (supplement), issued 1985, BERNSTEIN, "Cromolyn Sodium", pages 68S-73S.
- DIABETES, Volume 37, issued February 1988, DAMGE et al., "New Approach for Oral Administration of Insulin with Polyalkylcyanoacrylate Nanocapsules as Drug Carrier", pages 246-251.

## Description

### Field of the Invention

This invention relates to an oral delivery system, and in particular to salicyloyl phenylalanine for use as a delivery system for desferrioxamine, insulin and cromolyn sodium. The salicyloyl phenylalanine releasably encapsulates active agents and is suitable for oral administration to mammals. Methods for the preparation of such amino acids are also disclosed.

### Background of the Invention

Conventional means for delivering pharmaceutical and therapeutic agents to mammals often are severely limited by chemical or physical barriers or both, which are imposed by the body. Oral delivery of many biologically-active agents would be the route of choice if not for the presence of chemical and physicochemical barriers such as extreme pH in the gut, exposure to powerful digestive enzymes, and impermeability of gastrointestinal membranes to the active ingredient. Among the numerous pharmacological agents which are known to be unsuitable for oral administration are biologically active peptides and proteins, such as insulin. These agents are rapidly destroyed in the gut by acid hydrolysis and/or by proteolytic enzymes.

Prior methods for orally administering vulnerable pharmacological agents have relied on co-administration of adjuvants (e.g. resorcinols and non-ionic surfactants such as polyoxyethylene oleyl ether and n-hexadecyl polyethylene ether) to artificially increase the permeability of the intestinal walls; and co-administration of enzymatic inhibitors (e.g. pancreatic trypsin inhibitor, diisopropylfluorophosphate (DFF) and trasylol) to avoid enzymatic degradation. Liposomes as drug delivery systems for insulin and heparin have also been described. See, for instance, U.S. Patent No. 4,239,754; Patel et al. (1976) FEBS Letters Vol. 62, page 60; and Hashimoto et al. (1979) Endocrinol. Japan, Vol. 26, page 337. The broader use of the aforementioned methods, however, as drug delivery systems are precluded for reasons which include: (1) the use of toxic amounts of adjuvants or inhibitors; (2) narrow range of low MW cargoes; (3) poor stability of the system and inadequate shelf life; (4) difficulty in manufacturing; and (5) the failure of the method to adequately protect the active ingredient or promote its absorption.

More recently, artificial amino acid polymers or proteinoids forming microspheres have been described for encapsulating pharmaceuticals. For example, U.S. Patent No. 4,925,673 (the '673 patent), the disclosure of which is hereby incorporated by reference in its entirety, describes such microsphere constructs as well as methods for their preparation and use. The proteinoid microspheres of the '673 patent are useful for encapsulating a number of active agents, however the preparation methods result in a complex mixture of high molecular weight (MW) (> 1000 daltons) and low MW (≤ 1000 daltons) peptide-like polymers which are difficult to separate and yield relatively small amounts of the low MW microsphere-forming fraction. The UK patent application GB 2095994 discloses N-acyl amino acid derivatives and N-acyl peptide derivatives in combination with a medically active compound e.g. insulin for improving membrane permeability. US patent application No. 08/051019 (the earliest priority document of WO-A-9423767) discloses the use of modified amino acids for encapsulating active agents. Neither document discloses or suggests the use of salicyloylphenylalanine as a carrier for desferrioxiamine, insulin or cromolyn sodium.

Thus, there is a need in the art for a simple and inexpensive delivery system which is simple to prepare and which can encapsulate active agents such as proteinaceous drugs.

### Summary of the Invention

Compositions for orally delivering biologically-active agents incorporating modified amino acids, as carriers are provided. These compositions comprise
(a) a biologically active agent selected from the group consisting of desferrioxamine, insulin and cromolyn sodium (sodium or disodium cromoglycate) ;
(b) and an acylated amino acid carrier, being salicyloyl phenylalanine

Also described is a method for preparing these compositions which comprises mixing at least one biologically active agent with at least one carrier as described above and, optionally, a dosing vehicle.

In an alternative embodiment, the non-toxic carrier is orally administered to animals as part of a delivery system by blending or mixing the carrier with the biologically active agent prior to administration. The carrier may also form microspheres in the presence of the active agent. The microspheres containing the active agent are then orally administered. Also contemplated by the present invention are dosage unit forms that include these compositions.

As described herein salicyloyl phenylalanine is prepared by reacting phenylalanine with an acylating agent which reacts with free amino moieties present in the amino acid to form amides.

The modified amino acid is then recovered from the mixture.

The salicyloyl phenylalanine is non-toxic and can be orally administered to mammals as a drug delivery system by simply mixing the modified amino acid with an active agent prior to administration. Alternatively, the modified amino acid may be converted into microspheres in the presence of the active agent. The microspheres, containing encapsulated active agent, are then orally administered.

### Description of the Drawings

Figure 2 illustrates the induction of iron excretion in Cebus monkeys following oral administration of encapsulated versus unencapsulated desferrioxamine (DFO).

Figure 3 shows the iron excretion in rats following oral administration of encapsulated DFO.

Figure 4 to Figure 8 are graphic illustrations of the results of oral gavage testing in rats with insulin and salicyloyl phenylalanine carrier.

Figure 9 is a graphic illustration of the results of intraduodenal injection testing in rats with insulin and salicyloyl phenylalanine carrier.

Figures 10 and 11 are graphic illustrations of the results of oral gavage testing in rats with disodium cromoglycate and salicyloyl phenylalanine carrier.

### Detailed Description of the Invention

The present invention arose from the discovery that phenylalanine, in the modified form of salicyloyl-phenylalanine may be used to orally deliver sensitive bioactive agents, e.g. peptide hormones such as insulin, which would not be considered orally administrable due to their sensitivity to the denaturing conditions of the gastrointestinal (GI) tract. The modified amino acid, salicyloyl-phenylalanine may be used as a carrier by mixing it with the active agent or may be converted into microspheres containing encapsulated bioactive agent. In contrast to the modified amino acid of the invention, unmodified free amino acids provide inadequate protective effect for labile bioactive agents against degradation in the GI tract.

Pharmaceutical compositions containing insulin are useful for mammals suffering with diabetes.

Pharmaceutical compositions containing sodium cromolyn, an antiallergic, are useful for mammals suffering from respiratory afflictions, such as asthma or hay fever.

Other advantages provided by the present invention include the use of readily available and inexpensive starting materials and a cost-effective method for preparing and isolating modified amino acids which is simple to perform and is amenable to industrial scale-up production.

The modified amino acid, salicyloyl phenylalanine, of the present invention may be prepared by reacting the single amino acid, or amino acid esters with an amine modifying agent which reacts with free amino moieties present in the amino acids to form amides. Amino acid and amino acid esters are readily available from a number of commercial sources such as Aldrich Chemical Co. (Milwaukee, WI, USA); Sigma Chemical Co. (St. Louis, MO, USA) ; and Fluka Chemical Corp. (Ronkonkoma, NY, USA).

In practicing the invention, the amino acid is dissolved in aqueous alkaline solution of a metal hydroxide, e.g., sodium or potassium hydroxide, and heated at a temperature ranging between about 5°C and about 70°C, preferably between about 10°C and about 40°C, for a period ranging between about 1 hour and about 4 hours, preferably about 2.5 hours. The amount of alkali employed per equivalent of NH₂ groups in the amino acids generally ranges between about 1.25 and about 3 mmole, preferably between about 1.5 and about 2.25 mmole per equivalent of NH₂. The pH of the solution generally ranges between about 8 and about 13, preferably ranging between about 10 and about 12.

Thereafter, an amino modifying agent is then added to the amino acid solution while stirring. The temperature of the mixture is maintained at a temperature generally ranging between about 5°C and about 70°C, preferably between about 10°C and about 40°C, for a period ranging between about 1 and about 4 hours. The amount of amino modifying agent employed in relation to the quantity of amino acids is based on the moles of total free NH₂ in the amino acids. In general, the amino modifying agent is employed in an amount ranging between about 0.5 and about 2.5 mole equivalents, preferably between about 0.75 and about 1.25 equivalents, per molar equivalent of total NH₂ groups in the amino acids.

Suitable, but non-limiting, examples of amino modifying agents useful in practicing the present invention include acylating agents such as salicyloyl chloride and carbodiimide derivatives of phenylalanine.

The reaction is quenched by adjusting the pH of the mixture with a suitable acid, e.g., concentrated hydrochloric acid, until the pH reaches between about 2 and i about 3. The mixture separates on standing at room temperature to form a transparent upper layer and a white or off-white precipitate. The upper layer is discarded and modified amino acids are collected from the lower layer by filtration or decantation. The crude modified amino acids are then dissolved in water at a pH ranging between about 9 and about 13, preferably between about 11 and about 13. Insoluble materials are removed by filtration and the filtrate is dried in vacuo. The yield of modified amino acids generally ranges between about 30 and about 60%, and usually about 45%.

If desired, amino acid esters, e.g. methyl or ethyl esters of amino acids, may be used to prepare the modified amino acid of the invention. The amino acid esters, dissolved in a suitable organic solvent such as dimethylformamide or pyridine, are reacted with the amino modifying agent at a temperature ranging between about 5°C and about 70°C, preferably about 25°C, for a period ranging between about 7 and about 24 hours. The amount of amino modifying agents used relative to the amino acid esters are the same as described above for amino acids.

Thereafter, the reaction solvent is removed under negative pressure and the ester functionality is removed by hydrolyzing the modified amino acid ester with a suitable alkaline solution, e.g. IN sodium hydroxide, at a temperature ranging between about 50°C and about 80°C, preferably about 70°C, for a period of time sufficient to hydrolyze off the ester group and form the modified amino acid having a free carboxyl group. The hydrolysis mixture is then cooled to room temperature and acidified, e.g. aqueous 25% hydrochloric acid solution, to a pH ranging between about 2 and about 2.5. The modified amino acid precipitates out of solution and is recovered by conventional means such as filtration or decantation.

The modified amino acid may be purified by recrystallization or by fractionation on solid column supports. Suitable recrystallization solvent systems include acetonitrile, methanol and tetrahydrofuran. Fractionation may be performed on a suitable solid column supports such as alumina, using methanol/n-propanol mixtures as the mobile phase; reverse phase column supports using trifluoroacetic acid/acetonitrile mixtures as the mobile phase; and ion exchange chromatography using water as the mobile phase. When anion exchange chromatography is performed, a subsequent 0-500 mM sodium chloride gradient is employed. The modified amino acids may also be purified by extraction with a lower alcohol such as methanol, butanol, or isopropanol to remove low molecular weight non-sphere making material.

The modified amino acid of the present invention is soluble in alkaline aqueous solution (pH≥ 9.0) ; partially soluble in ethanol, n-butanol and 1:1 (v/v) toluene/ethanol solution and insoluble in neutral water. The titratable functional groups remaining in the modified amino acids are as follows: carboxylic acid groups (COOH) generally ranging between about 1.5 and about 3.5 milliequivalents/g, preferably about 2.3 milliequivalents/g; and amino groups (NH₂) generally ranging between about 0.3 and about 0.9 milliequivalents/g, preferably about 0.5 milliequivalents/g.

The modified amino acid may be used as a drug delivery carrier by simply mixing the modified amino acid with the active ingredient prior to administration. Alternatively, the modified amino acid may be used to form encapsulating microspheres containing the active agent. The modified amino acid of the invention is particularly useful for the oral administration of certain pharmacological agents, e.g., small peptide hormones, which, by themselves, pass slowly or not at all through the gastrointestinal mucosa and/or are susceptible to chemical cleavage by acids and enzymes in the gastrointestinal tract.

If the modified amino acid is used as a carrier for an active ingredient, an aqueous solution of modified amino acids is mixed with an aqueous solution of the active ingredient just prior to administration. The solutions may contain additives such as phosphate buffer salts, citric acid, acetic acid, gelatin and gum acacia.

A solution of the modified amino acid is prepared by mixing the amino acid in aqueous solution in an amount ranging between about 1 mg and about 2000 mg, preferably ranging between about 300 mg and about 800 mg per mL of solution. The mixture is then heated to a temperature ranging between about 20 and about 50°C, preferably about 40°C, until the modified amino acid is dissolved. The final solution contains between about 1 mg and about 2000 mg of modified amino acids per mL of solution, preferably between about 300 and about 800 mg per mL. The concentration of active agent in the final solution varies and is dependent on the required dosage for treatment.

The modified amino acid may be used to prepare microspheres for encapsulating active agents. A useful procedure is as follows: Modified amino acid is dissolved in deionized water at a concentration ranging between about 75 and about 200 mg/ml, preferably about 100 mg/ml at a temperature between about 25°C and about 60°C, preferably about 40 C. Particulate matter remaining in the solution may be removed by conventional means such as gravity filtration over filter paper.

Thereafter, the amino acid solution, maintained at a temperature of about 40 C, is mixed 1:1 (V/V) with an aqueous acid solution (also at about 40 C) having an acid concentration ranging between about 0.05 N and about 2 N, preferably about 1.7 N. The resulting mixture is further incubated at 40 C for a period of time effective for microsphere formation as observed by light microscopy. In practicing this invention, the preferred order of addition is to add the amino acid solution to the aqueous acid solution.

Suitable acids include any acid which does not (a) adversely effect the modified amino acids, e.g., chemical decomposition; (b) interfere with microsphere formation; (c) interfere with microsphere encapsulation of cargo; and (d) adversely interact with the cargo. Preferred acids for use in this invention include acetic acid, citric acid, hydrochloric acid, phosphoric acid, malic acid and maleic acid.

In practicing the invention, a microsphere stabilizing additive preferably incorporated into the aqueous acid solution or into the amino acid solution, prior to the microsphere formation process. The presence of such additives promotes the stability and dispersibility of the microspheres in solution.

The additives may be employed at a concentration ranging between about 0.1 and 5 % (W/V), preferably about 0.5 % (W/V). Suitable, but non-limiting, examples of microsphere stabilizing additives include gum acacia, gelatin, polyethylene glycol, and polylysine.

Under these conditions, the modified amino acid molecules form hollow microspheres of less than 10 microns in diameter. If the modified amino acid microspheres are formed in the presence of a soluble material, e.g., a pharmaceutical agent in the aforementioned aqueous acid solution, this material will be encapsulated in the hollows of the microspheres and confined within the amino acid wall defined by the spherical structure. In this way, one can encapsulate pharmacologically active materials such as peptides, proteins, and polysaccharides as well as charged organic molecules, e.g., antimicrobial agents, having poor bioavailability by the oral route. The amount of pharmaceutical agent which may be encapsulated by the microsphere is dependent on a number of factors which include the concentration of agent in the encapsulating solution, as well as the affinity of the cargo for the carrier.

The modified amino acid microspheres of the invention are pharmacologically harmless and do not alter the physiological and biological properties of the active agent. Furthermore, the encapsulation process does not alter the pharmacological properties of the active agent. While any pharmacological agent can be encapsulated within the amino acid microspheres, it is particularly valuable for delivering chemical or biological agents which otherwise would be destroyed or rendered less effective by conditions encountered within the body of the mammal to which it is administered, before the microsphere reaches its target zone (i.e., the area in which the contents of the microsphere are to be released) and which are poorly absorbed in the gastrointestinal tract.

The particle size of the microsphere plays an important role in determining release of the active agent in the targeted area of the gastrointestinal tract. Microspheres having diameters between about ≤ 0.1 microns and about 10 microns, preferably between about 5.0 microns and about 0.1 microns, and encapsulating active agents are sufficiently small to effectively release the active agent at the targeted area within the gastrointestinal tract. Small microspheres can also be administered parenterally by being suspended in an appropriate carrier fluid (e.g., isotonic saline) and injected into the circulatory system or subcutaneously. The mode of administration selected will, of course, vary, depending upon the requirement of the active agent being administered. Large amino acid microspheres (>10 microns) tend to be less effective as oral delivery systems.

The size of the microspheres formed by contacting modified amino acid with water or an aqueous solution containing active agents can be controlled by manipulating a variety of physical or chemical parameters, such as the pH, osmolarity or ionic strength of the encapsulating solution, and by the choice of acid used in the encapsulating process.

The amino acid microspheres of the invention may be orally administered alone as solids in the form of tablets, pellets, capsules, and granulates suitable for suspension in liquids such as water or edible oils. Similarly, the microspheres can be formulated into a composition containing one or more physiologically compatible carriers or excipients, and which can be administered via the oral route. These compositions may contain conventional ingredients such as gelatin, polyvinylpyrrolidone and fillers such as starch and methyl cellulose. Alternatively, small microspheres (size less than 10 µm) can be administered via the parenteral route.

The amount of active agent in the composition typically is a pharmacologically or biologically effective amount. However, the amount can be less than a pharmacologically or biologically effective amount when the composition is used in a dosage unit form, such as a capsule, a tablet or a liquid, because the dosage unit form may contain a multiplicity of carrier/biologically-active agent compositions or may contain a divided pharmacologically or biologically effective amount. The total effective amounts will be administered by cumulative units containing in total pharmacologically or biologically active amounts of biologically-active agent.

The total amount of biologically-active agent to be used can be determined by those skilled in the art. However, it has surprisingly been found that with certain biologically-active agents, the use of the presently disclosed carriers provides extremely efficient delivery. Therefore, lower amounts of biologically-active agent than those used in prior dosage unit forms or delivery systems can be administered to the subject, while still achieving the same blood levels and therapeutic effects.

The amount of carrier in the present composition is a delivery effective amount and can be determined for any particular carrier or biologically-active agent by methods known to those skilled in the art.

Dosage unit forms can also include any of excipients; diluents; disintegrants; lubricants; plasticizers; colorants; and dosing vehicles, including, but not limited to water, 1,2-propane diol, ethanol, olive oil, or any combination thereof.

Administration of the present compositions or dosage unit forms is oral or by intraduodenal injection.

The following examples are illustrative of the invention but are not intended to limit the scope of the invention.

### Example 8: Modification of Phenylalanine Using Acetylsalicyloyl Chloride

10 ml of pyridine were added to a mixture of acetylsalicyloyl chloride (10 g., 50.4 mmol) and phenylalanine benzyl ester toluene sulfonate (21.4 g., 50 mmol). The reaction was allowed to stir overnight and was monitored by thin layer chromatography (TLC). The reaction mixture was washed with IN hydrochloric acid (100 ml). The organic layer was separated, dried with MgSO₄, filtered, and concentrated to provide an oil. The oil was purified by flash chromatography on silica gel (30% EtOAc/hexane) to provide 16.9 g (80.9%) of pure diester product, m.p. 56-57°C, Rf = 0.35 (20% EtOAc/hexane).

The diester, acetylsalicyloyl phenylalanine benzyl ester (30 g. 71.9 mmol), was added to an aqueous saturated solution of NaHCO₃ (50 ml), acetone (200 ml), and methanol (125 ml). This mixture was stirred at room temperature and was monitored by TLC until no starting diester remained. Removal of the solvents *in vacuo* provided an oily suspension which was extracted with methylene chloride (3 x 80 ml). The extracts were combined, washed with water, and dried with MgSO₄. The dried extracts were filtered and concentrated to provide the benzyl ester product which was purified by flash chromatography on silica gel (30% EtOAc/hexane).

The salicyloyl phenylalanine benzyl ester (16.9 g, 45 mmol) was placed in methanol (400 ml) in a reaction vessel, and a palladium/carbon catalyst was added. The vessel was flushed with nitrogen (3 times), and hydrogen gas (1.7 g) was introduced. The reaction was monitored by TLC and was complete in about 4 hours. The catalyst was removed by filtration and washed with methanol. Removal of the solvent provided a quantitative yield of the pure salicyloyl phenylalanine, m.p. 52-53°C.

Properties are listed below:
¹H NMR (300 MHz, DMSO-d6) δ: 13 (s, 1H), 12 (d, 1H), 9 (d, 1H), 8 (dd, 1H), 7.4 (m, 1H), 7.2 (q, 5H), 6.9 (t, 2H), 4.7 (m, 1H), 3.1 (m, 2H).

| | | | |
|---|---|---|---|
| Analysis: calculated for C₁₆H₁₅NO₄ | C,67.36; | H,5.30; | N,4.91; |
| Found | C,67.15; | H,5.27; | N,4.84; |

### Example 9: Preparation and Evaluation of Desferrioxamine (DFO)-containing Microspheres

In this Example, a study was undertaken to evaluate the relative efficacy of the iron chelator desferrioxamine B (DFO) when administered orally with modified amino acid carriers. Currently, DFO must be administered parenterally in order to treat iron overload in mammals. The currently accepted mode of treatment (subcutaneous infusion of DFO) raises problems of compliance, especially in patients who must be maintained on chelation therapy for long periods of time, and is also difficult to achieve in Third World countries with suboptimal medical facilities. Thus, there is a need for a convenient orally administrable form of DFO. Effective amounts of DFO are well known in the art and range between about 10 and about 50 mg/kg/day (or between about 0.4 to about 2.0 g/day total.)

Preparation of DFO in modified amino acid carriers was performed in the same manner as described above. DFO was obtained from Ciba-Geigy (Basel, Switzerland) under the trade name DESFERAL. DFO modified amino acid carriers were prepared with salicyloyl-Phe modified amino acid, and the final modified amino acid carrier suspension contained 125 mg/ml DFO.

The efficacy of modified amino acid-carried DFO was evaluated in both the Cebus monkey iron clearance model system (Bergeron et al., Sixth Cooley's Anemia Symposium, Ann.N.Y.Acad.Sci. 612:378 (1991)), and in bile duct-cannulated rats (Bergeron et al., Blood 79:1882 (1992)).

### Experimental Procedure

### A. Cebus monkeys:

Eight days prior to drug administration, Cebus monkeys were anesthetized with Ketamine (7-10 mg/kg intramuscularly), after which a baseline blood sample was obtained. The animals were then transferred to metabolic cages and started on a low-iron liquid diet. One day prior to administration, the animals were fasted. On day 0, the animals were anesthetized and the drug was administered at a dose of 200 mg/kg using a pill gun. An additional blood sample was obtained on day 5.

Fecal and urine samples were collected at 24-h intervals between day -3 and day +5. The volume of the urine and the wet weight of the feces were determined, after which the samples were autoclaved. The urine samples were then acidified with low-iron nitric acid. The feces samples were freeze-dried for 48h, after which their dry weight was measured; they were then refluxed for 48h with low-iron nitric acid. The iron content of the acidified urine and feces samples was then measured in triplicate on an atomic absorption spectrophotometer. The iron clearance rates were calculated on the basis of a 1:1 DFO-iron complex. The iron output for 4 days prior to administration of the drug was averaged and subtracted from the iron clearance after administration; this value was then divided by the theoretical output to calculate the efficiency of clearance.

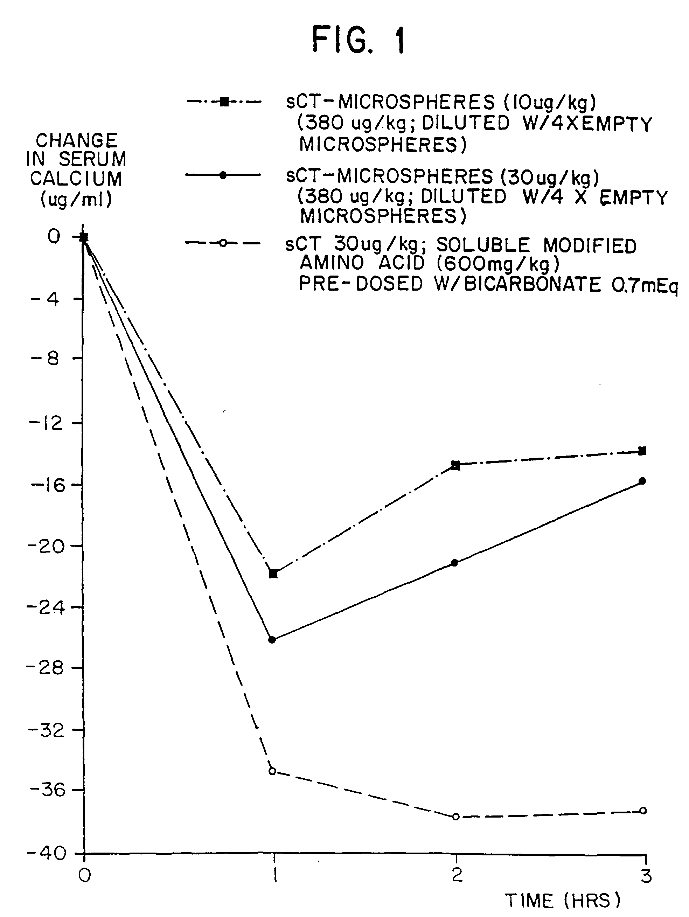

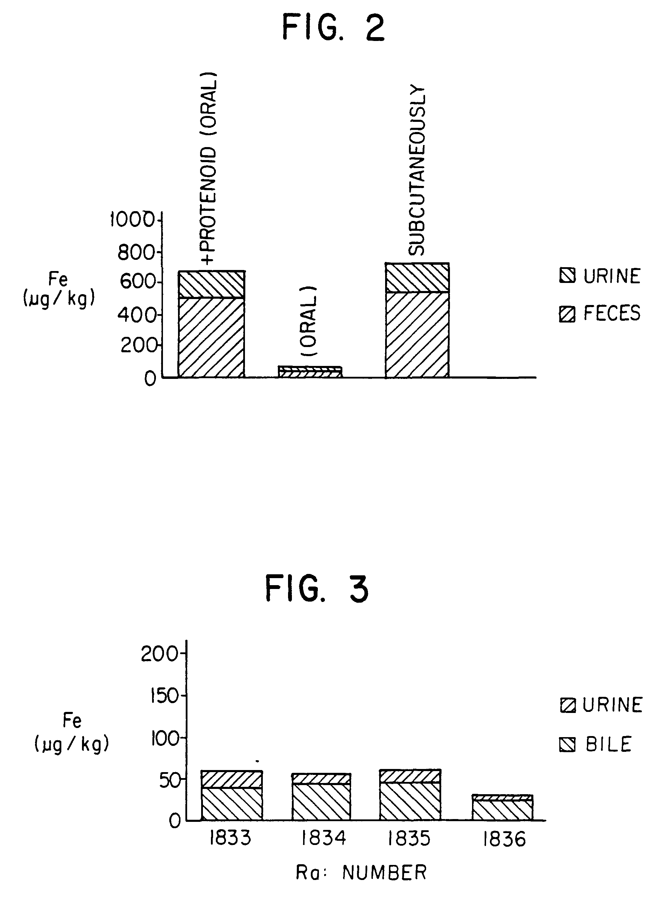

Oral administration of DFO in the absence of modified amino acid carriers induced little if any clearance of iron in a 48-h period following administration (Figure 2) In contrast, DFO prepared with the modified amino acid carriers of the present invention prior to administration induced a rapid peak of iron secretion in both urine and feces, corresponding to a 4% clearance rate during that interval. Subcutaneously administered DFO induced a 5% clearance rate. These results demonstrate that a single dose of DFO modified amino acid carrier was substantially as effective as parenterally administered DFO in delivering a therapeutically active bioavailable dose of DFO.

### B. Rats

Male Sprague-Dawley rats were anesthetized, after which their bile ducts were cannulated, such that continuous bile samples could be collected while the animals were free to move around their cages. The rats were fasted for 24h, after which the drug was administered by gavage at a dose of 45 mg/kg. Bile samples were collected at 3-h intervals, and urine samples were taken every 24 h. The iron content of the bile and urine samples were measured by atomic absorption spectrometry essentially as described in part A.

Figure 3 shows the biliary and urinary excretion of iron in rats after oral administration of DFO with modified amino acid carriers. The stimulation of iron excretion by orally administered modified amino acid-carried DFO is equivalent to that observed when free DFO was administered via a subcutaneous route (Bergeron et al., Blood, ibid.).

### Example 10: Preparation of Insulin/Delivery System

In a test tube, 1800 mg of salicyloyl phenylalanine carrier were added to 4 ml of water. The solution was stirred, and the pH was adjusted to 8.0-8.5 with NaOH (1.0N) or HCl (1.0N).

Insulin was prepared by adding 20 mg of insulin to 2 mL of NaHCO₃ solution (40 mg/ml NaHCO₃). The concentration of insulin was 10 mg/ml.

0.900 ml of insulin solution was added to the carrier solution. Water was added to bring the total volume to 6.0 mL. The sample had a carrier concentration of 300 mg/mL. The total insulin concentration was 1.5 mg/mL.

### Example 11 Insulin In Vivo Experiments in Rats

Following the procedure in Example 10, insulin/carrier preparations of 3 mg/kg of insulin mixed with 60 mg/kg of salicyloyl-Phe carrier, 600 mg/kg of salicyloyl-Phe carrier and 1200 mg/kg of salicyloyl-Phe carrier, respectively were prepared. Fasted rats were anesthetized with Ketamine (14 mg/kg). The fasted rats were administered, by oral gavage, a dosing volume of two (2) mL/kg of one of the preparations. Blood samples were collected by cutting the tip of the tail, and one drop of blood was analyzed with a ONE TOUCH II Glucose Analyzer (available from Life Scans, Inc., Mitpitsas, CA, USA).

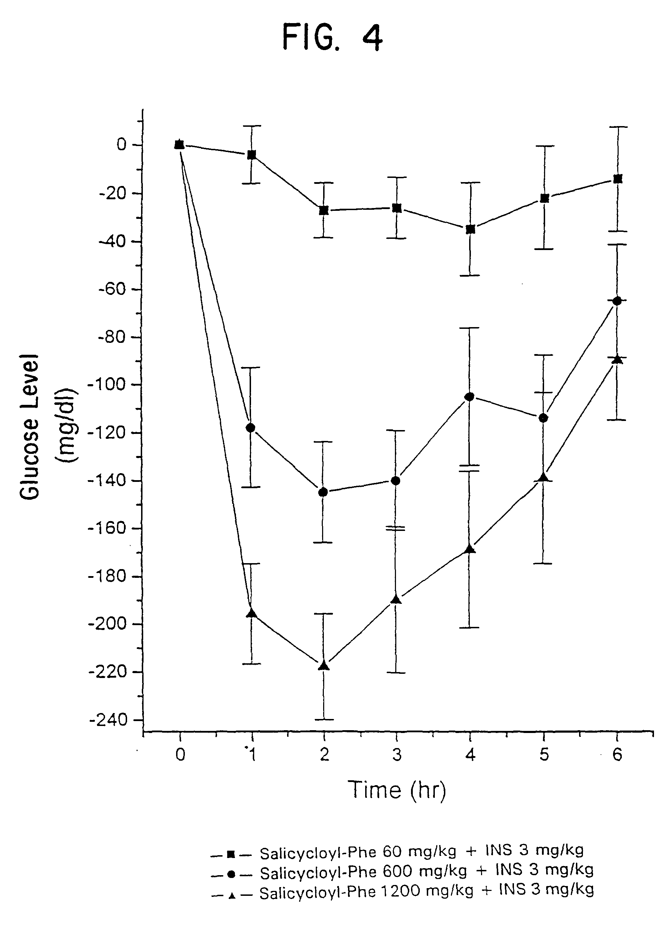

Results are illustrated in Figure 4.

### Example 12 Insulin In Vivo Experiment

Following the procedure in Example 10, insulin/carrier preparations were prepared, and a first group of fasted rats was administered, by oral gavage, 0.5 mg/kg of insulin mixed with 600 mg/kg of salicyloyl-Phe carrier. A second group of fasted rats was administered 600 mg/kg of salicyloyl-Phe carrier without insulin. Blood samples were collected and analyzed as described in Example 11.

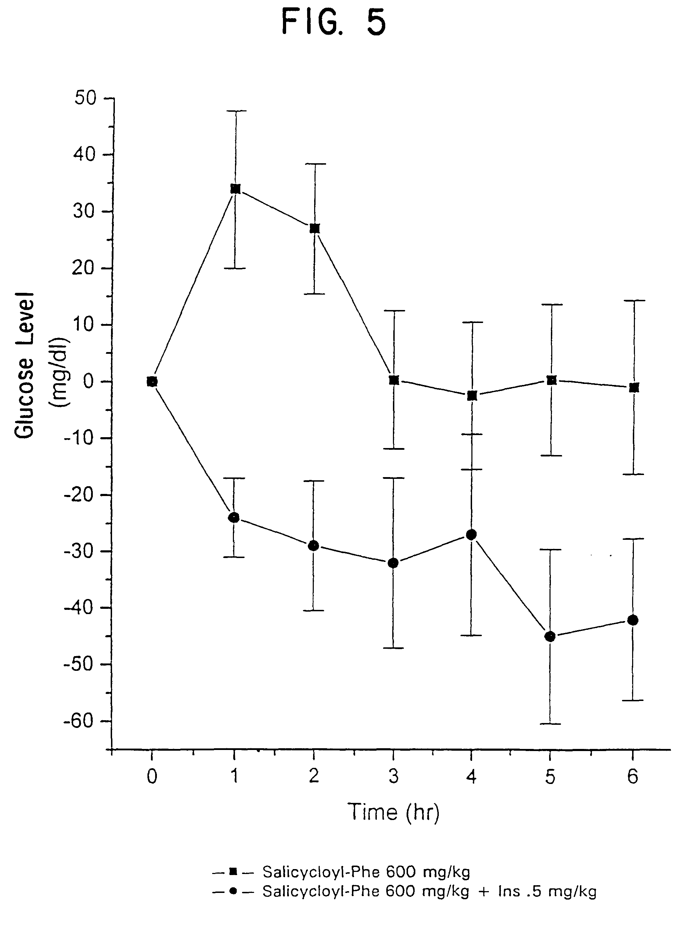

Results are illustrated in Figure 5.

### Example 13 Insulin In Vivo Experiment

Following the procedure in Example 10, insulin/carrier preparations were prepared, and a first group of fasted rats was administered, by oral gavage, 0.5 mg/kg of insulin mixed with 600 mg/kg of salicyloyl-Phe carrier. A second group of fasted rats was administered 3 mg/kg of insulin without a carrier. Blood samples were collected and analyzed as described Example 11.

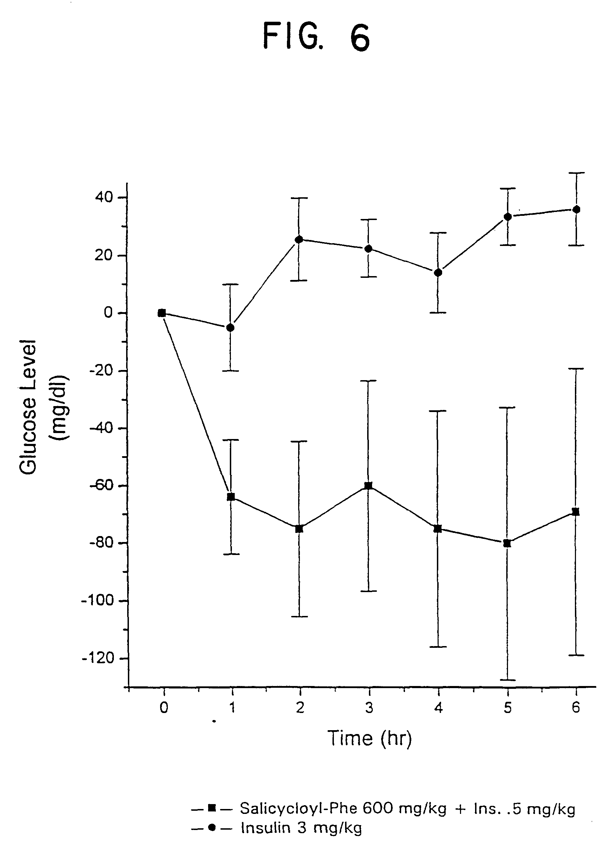

Results are illustrated in Figure 6.

### Example 14 Insulin In Vivo Experiment

Following the procedure in Example 10, insulin carrier preparations were prepared, and a first group of fasted rats was administered, by oral gavage, 1.0 mg/kg of insulin mixed with 600 mg/kg of salicyloyl-Phe carrier. A second group of fasted rats was administered 600 mg/kg of salicyloyl-Phe carrier without insulin. Blood samples were collected an analyzed as described in Example 11.

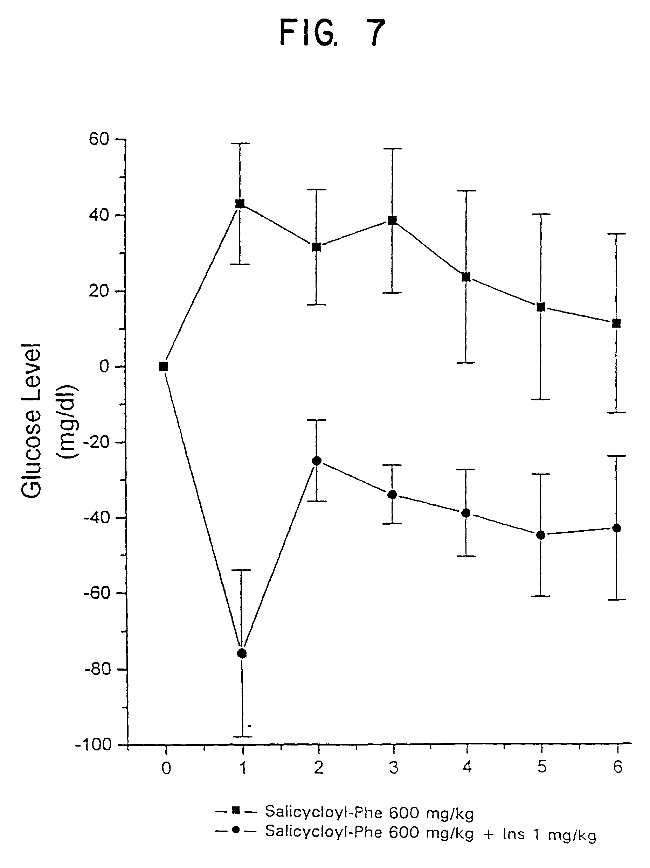

Results are illustrated in Figure 7.

### Example 15 Insulin In Vivo Experiment

Following the procedure in Example 10, insulin/carrier preparations were prepared, and a first group of fasted rats was administered, by oral gavage, 3.0 mg/kg of insulin mixed with 1200 mg/kg of salicyloyl-Phe carrier. A second group of fasted rats was administered, by oral gavage, doses of 1200 mg/kg of salicyloyl-Phe carrier without insulin. Blood samples were collected and analyzed as described in Example 11.

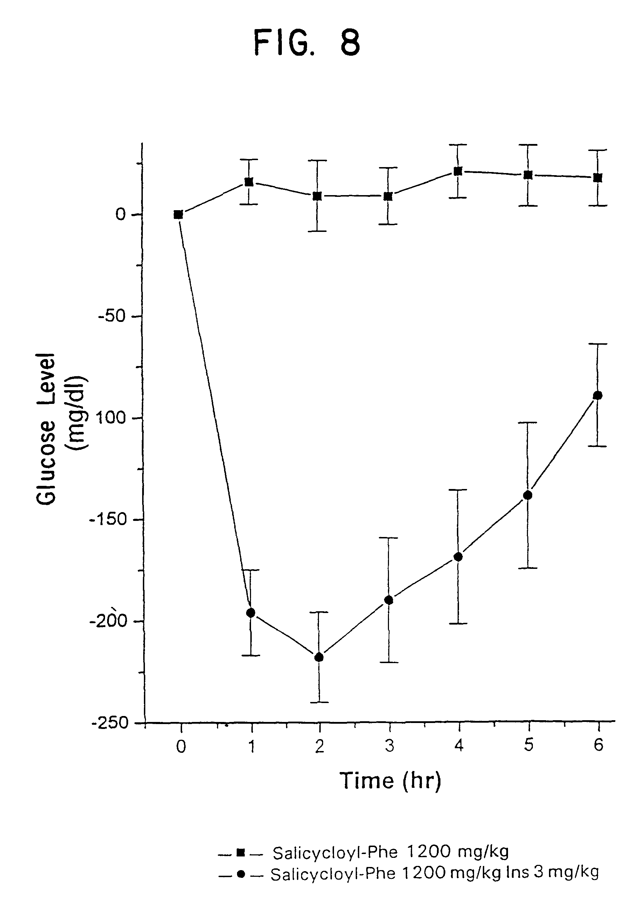

Results are illustrated in Figure 8.

### Example 16 In Vivo Insulin Experiment

Following the procedure in Example 10, insulin/carrier preparations were prepared, and a first group of fasted rats was administered, by intraduodenal injection, 1.0 mg/kg of insulin mixed with 300 mg/kg of salicyloyl-Phe carrier. A second group of rats was administered, by intraduodenal injection, 1.0 mg/kg of insulin mixed with 300 mg/kg of salicyloyl-Phe carrier and 6 mg/kg of urea. Blood samples were collected and analyzed as described in Example 11.

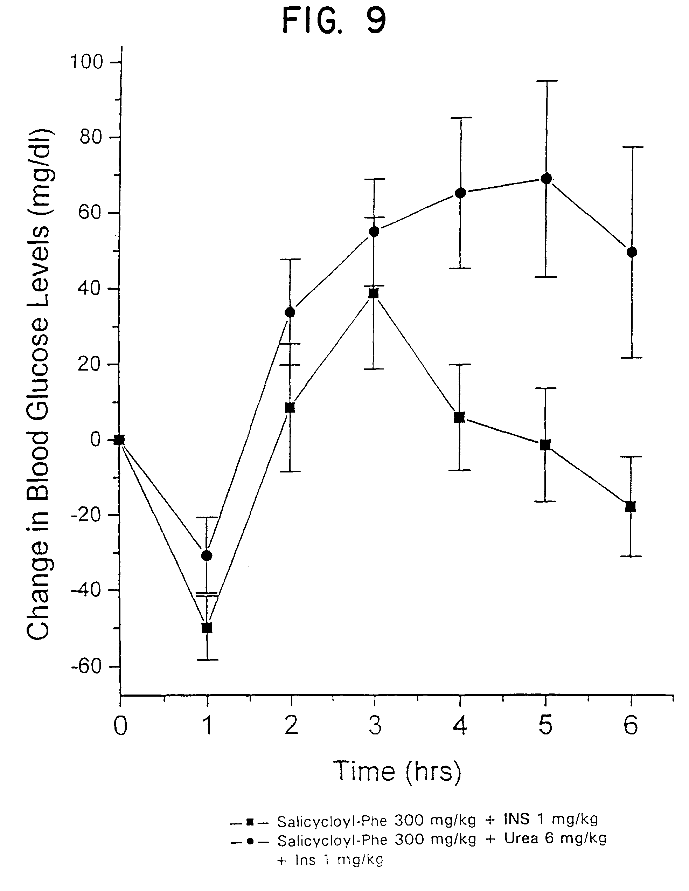

Results are illustrated in Figure 9.

### Example 17

### In Vivo Evaluation of Cromoglycate Preparations in Rats

A preparation of 100 mg/ml salicyloyl-Phe solution in 0.85N citric acid and 0.5% acacia and 25 mg/ml of disodium cromoglycate was prepared.

Fasted rats were administered, by oral gavage, dosages of the preparation containing 50 mg/kg of disodium cromoglycate mixed with 200 mg/kg of carrier, at a dosing volume of two (2) mL/kg. The delivery was evaluated by using the procedure described by A. Yoshimi in *Pharmcobio-Dyn.,* **15,** pages 681-686, (1992).

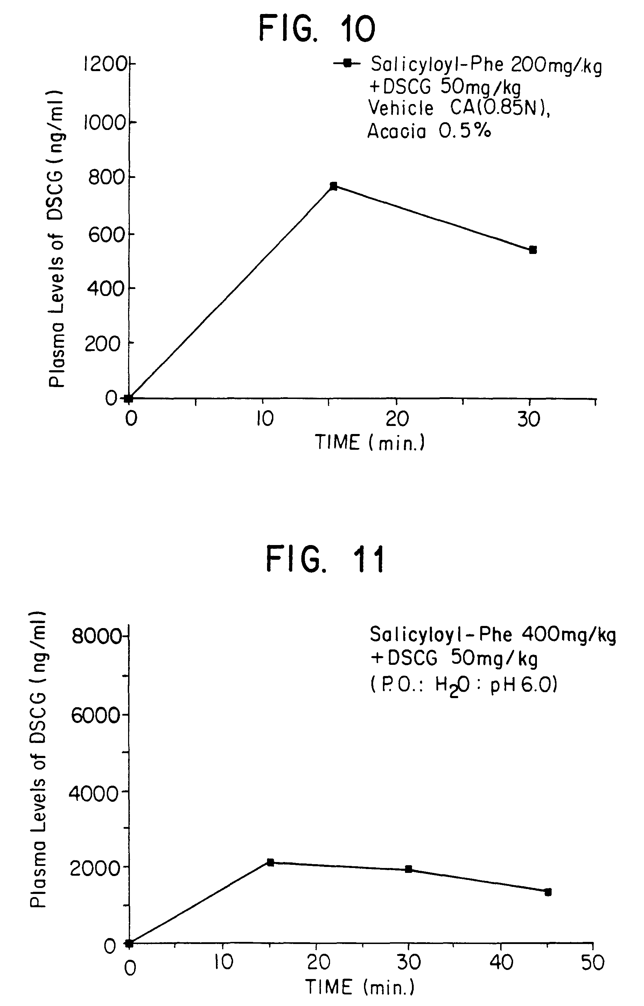

Results are illustrated in Figure 10.

### Example 18

### In Vivo Evaluation of Cromoglycate Preparations in Rats

Following the procedure in Example 17, a disodium cromoglycate preparation was prepared, fasted rats were administered, by oral gavage, a mixture of 50 mg/kg of disodium cromoglycate and 400 mg/kg of salicyloyl-Phe carrier, at a dosing volume of two (2) mL/kg.

Results are illustrated in Figure 11.

As clearly illustrated by the data in the Examples and Figures the use of compositions of the subject invention show significant advantages for the delivery of biologically active agents.

## Claims

1. A composition of matter comprising
(a) a biologically active agent selected from the group consisting of desferrioxamine, insulin and cromolyn sodium; and
(b) an acylated amino acid carrier being salicyloylphenylalanine.

2. The composition as defined in claim 1 wherein said biologically active agent is desferrioxamine.

3. The composition as defined in claim 1 wherein said biologically active agent is insulin.

4. The composition as defined in claim 1 wherein said biologically active agent is cromolyn sodium.

5. A pharmaceutical composition comprising;
(a) a biologically active agent selected from the group consisting of desferrioxamine, insulin and cromoyln sodium;
(b) an acylated amino acid carrier being salicyloylphenylalanine; and
(c) a pharmaceutically acceptable carrier or diluent.

6. The pharmaceutical composition as defined in claim 5 wherein said biologically active agent is desferrioxamine.

7. The pharmaceutical composition as defined in claim 5 wherein said biologically active agent is insulin.

8. The pharmaceutical composition as defined in claim 5 wherein said biologically active agent is cromolyn sodium.

9. Use of a composition as defined in claim 1 or 2 for preparing a medicament in orally applicable form for lowering iron concentration in a mammal.

10. Use of a composition as defined in claim 1 or 3 for preparing a medicament in orally applicable form for treating diabetes in a mammal.

11. Use of a composition as defined in claim 1 or 4 for preparing a medicament in orally applicable form for treating respiratory afflications in a mammal.

12. A dosage unit form comprising
(A) a pharmaceutical composition as defined in claim 5; and
(B)
(a) an excipient,
(b) a diluent,
(c) a disintegrant,
(d) a lubricant,
(e) a plasticizer,
(f) a colorant,
(g) a dosing vehicle, or
(h) any combination thereof.

## Patentansprüche

1. Stoffzusammensetzung, umfassend
(a) eine biologisch aktive Substanz, ausgewählt aus der Gruppe bestehend aus Desferrioxamin, Insulin und Cromolyn-Natrium; und
(b) eine acylierte Aminosäure als Träger, wobei es sich um Salicyloylphenylalanin handelt.

2. Zusammensetzung wie in Anspruch 1 definiert, wobei die biologisch aktive Substanz Desferrioxamin ist.

3. Zusammensetzung wie in Anspruch 1 definiert, wobei die biologisch aktive Substanz Insulin ist.

4. Zusammensetzung wie in Anspruch 1 definiert, wobei die biologisch aktive Substanz Cromolyn-Natrium ist.

5. Pharmazeutische Zusammensetzung, umfassend:
(a) eine biologisch aktive Substanz, ausgewählt aus der Gruppe bestehend aus Desferrioxamin, Insulin und Cromolyn-Natrium;
(b) eine acylierte Aminosäure als Träger, wobei es sich um Salicyloylphenylalanin handelt; und
(c) einen pharmazeutisch annehmbaren Träger oder ein pharmazeutisch annehmbares Verdünnungsmittel.

6. Pharmazeutische Zusammensetzung wie in Anspruch 5 definiert, wobei die biologisch aktive Substanz Desferrioxamin ist.

7. Pharmazeutische Zusammensetzung wie in Anspruch 5 definiert, wobei die biologisch aktive Substanz Insulin ist.

8. Pharmazeutische Zusammensetzung wie in Anspruch 5 definiert, wobei die biologisch aktive Substanz Cromolyn-Natrium ist.

9. Verwendung einer Zusammensetzung wie in Anspruch 1 oder 2 definiert, zum Herstellen eines Arzneimittels in oral anwendbarer Form zum Absenken der Eisenkonzentration bei einem Säuger.

10. Verwendung einer Zusammensetzung wie in Anspruch 1 oder 3 definiert, zum Herstellen eines Arzneimittels in oral anwendbarer Form zum Behandeln von Diabetes bei einem Säuger.

11. Verwendung einer Zusammensetzung wie in Anspruch 1 oder 4 definiert, zum Herstellen eines Arzneimittels in oral anwendbarer Form zum Behandeln von Atembeschwerden bei einem Säuger.

12. Dosierungseinheitsform, umfassend
(A) eine pharmazeutische Zusammensetzung wie in Anspruch 5 definiert; und
(B) (a) ein Excipiens,
(b) ein Verdünnungsmittel,
(c) ein Zerfallhilfsmittel,
(d) ein Schmiermittel,
(e) einen Weichmacher,
(f) ein Färbemittel,
(g) ein Dosiervehikel oder
(h) eine beliebige Kombination davon.

## Revendications

1. Composition de matière comprenant :
(a) un agent biologiquement actif choisi dans le groupe formé par la deferoxamine, l'insuline et le cromolyne sodique ; et
(b) comme véhicule un aminoacide acylé qui est la salicyloylphénylalanine.

2. Composition selon la revendication 1, dans laquelle ledit agent biologiquement actif est la deferoxamine.

3. Composition selon la revendication 1, dans laquelle ledit agent biologiquement actif est l'insuline.

4. Composition selon la revendication 1, dans laquelle ledit agent biologiquement actif est le cromolyne sodique.

5. Composition pharmaceutique comprenant :
(a) un agent biologiquement actif choisi dans le groupe formé par la deferoxamine, l'insuline et le cromolyne sodique ;
(b) comme véhicule un aminoacide acylé qui est la salicyloylphénylalanine ; et
(c) un véhicule pharmaceutiquement acceptable ou un diluant.

6. Composition pharmaceutique selon la revendication 5, dans laquelle ledit agent biologiquement actif est la deferoxamine.

7. Composition pharmaceutique selon la revendication 5, dans laquelle ledit agent biologiquement actif est l'insuline.

8. Composition pharmaceutique selon la revendication 5, dans laquelle ledit agent biologiquement actif est le cromolyne sodique.

9. Utilisation d'une composition selon l'une des revendications 1 ou 2 pour la préparation d'un médicament administrable par voie orale pour diminuer la concentration de fer chez un mammifère.

10. Utilisation d'une composition selon l'une des revendications 1 ou 3 pour la préparation d'un médicament administrable par voie orale pour traiter les diabètes chez un mammifère.

11. Utilisation d'une composition selon l'une des revendications 1 ou 4 pour la préparation d'un médicament administrable par voie orale pour traiter les affections respiratoires chez un mammifère.

12. Dose unitaire comprenant :
(A) une composition pharmaceutique selon la revendication 5, et
(B)
(a) un excipient,
(b) un diluant,
(c) un désintégrant,
(d) un lubrifiant,
(e) un plastifiant,
(f) un colorant,
(g) un véhicule de dosage, ou
(h) une combinaison de ceux-ci.
